## Europäisches Patentamt
## European Patent Office
## Office européen des brevets

(19)

(11) Publication number: **0 222 712**
**B1**

(12)

# EUROPEAN PATENT SPECIFICATION

(45) Date of publication of the patent specification:
06.06.90

(51) Int. Cl.⁵: **A61K 47/00**

(21) Application number: 86830320.7

(22) Date of filing: 04.11.86

(54) Veterinary compositions containing antimicrobial agents, and their use.

(30) Priority: 08.11.85 IT 359585

(43) Date of publication of application:
20.05.87 Bulletin 87/21

(45) Publication of the grant of the patent:
06.06.90 Bulletin 90/23

(84) Designated Contracting States:
AT BE CH DE ES FR GB GR LI NL SE

(56) References cited:
EP-A- 0 058 015
FR-A- 2 265 408
US-A- 3 849 569
US-A- 4 049 830

(73) Proprietor: FATRO S.p.A., Via Emilia Levante, 317,
I-40064 Ozzano Emilia Bologna(IT)

(72) Inventor: Montecchi, Lauretta, Via Don Ercolai, 8,
I-40033 Casalcchio di Reno (Bologna)(IT)
Inventor: Verardi, Paolo, Via Seminario, 46, I-40068 San
Lazzaro di Savena (Bologna)(IT)

(74) Representative: Pederzini, Paolo, BUGNION S.p.A. Via
Farini n. 37, I-40124 Bologna(IT)

## Description

The invention relates to novel veterinary compositions that contain antibiotics, or antimicrobial agents generally, and are suitable for administration by intramammary injection. Such products serve in the prevention and cure of mastitis, and in particular, of bovine mastitis and mastitis in other ruminant species, during lactation and dry periods.

It is widely known that bovine mammary organs are especially vulnerable to infection from bacteria and other micro-organisms during lactation.

Hygiene and environmental factors, and metabolic disturbances deriving from high milk yield, combine to create conditions that favour the onset of such infections and oblige breeders to use antimastitic products, whether for prevention or cure; products of this general type contain antibiotics and other antimicrobial agents, and are administered by intramammary injection.

GB-A 1 441 747 sets forth the notion of injecting a compound into the teat of the animal, which contains an antibacterial agent and a metal salt held in a solid or semisolid medium that melts at body temperature.

EP-B 76068 discloses the idea of formulating a substance comprising a cyclohexane elastomer into which the anti-bacterial agent is incorporated.

Both products, however, are intended for use during the dry period, during which an attempt is made to avoid the onset of any infection or counter-infections, but not during lactation.

The prior art also embraces veterinary compounds for intramammary injection, used in the treatment of mastitis during lactation, which are formulated from antibiotics and other antimicrobial agents held in an oily medium such as vaseline oil or peanut oil, petroleum jelly, lanolin, beeswax, or aluminium monostearate and polysorbate.

These compounds, which are available for general purchase, occasion only a partial temporal release of the active constituent which produces therapeutic activity, so that the potential of the compound is diminished. When treating mastitis during lactation, in fact, it is common practice to adminster the intramammary immediately after milking in order to ensure its full dispersion before the next milking period, which will generally take place 12 hours later.

The object of the invention is that of setting forth novel veterinary compositions, employing antibiotics or chemotherapeuticals designed for the prevention and cure of mastitis, which are both suitable for intra mammary administratrion, and free from the drawbacks mentioned above.

The stated object is realized with a novel composition that contains antibiotics or chemotherapeuticals held in a suitable medium, and enables a reduction in the latency time lapse occurring between its administration and the subsequent manifestation of antimicrobial activity, by virtue of ensuring a fast and total release of the active constituent.

Furthermore, the rapid dispersion rate of the medium in milk permits of reducing the time the animal is suspended from milking following mastitis therapy, during the lactation period.

A veterinary composition for the prevention and cure of mastitis according to the invention is characterized in that it contains at least one antimicrobial agent dispersed in a mixture consisting of triglycerides of palmitic and stearic acid with polyoxyethylenated cetyl alcohol and stearyl alcohol, and dispersed in an oily medium of mineral, vegetable, synthetic or mixed extraction.

More exactly, a composition according to the invention consists of: an antimicrobial agent introduced, in the form of a salt, in an amount of between 2% (w/w) and 20% (w/w) a mixture of triglycerides of palmitic and stearic acid introduced at a rate of between 0.5% (w/w) and 20% (w/w) and polyoxyethylenated cetyl and stearyl alcohol introduced at a rate of between 0.5% (w/w) and 20% (w/w), all dispersed in an oily medium of mineral, vegetable, synthetic or mixed extraction.

The composition thus formulated possesses long-term stability, can easily be administered, and has markedly reduced side-effects.

It has been found, with a certain degree of surprise, that a composition according to the invention possesses a hydrophilic-lipophilic balance, expressed numerically as 'HLB', remarkably similar to that of milk, thus enabling its rapid and total dispersion and occasioning the release of a quantity of the active constituent quantifiable at over 80%, in less than 12 hours.

In addition, these features permit of a reduction in the length of time the animal remains suspended from milking following antimastitic treatment, during lactation.

The active constituent in question might be one of a number of prior art antimicrobial agents (or a combination of such agents) to be effective in combating udder infections.

Antimicrobial agents may belong to any one of the following groups: sulphamides, penicillin, cephalosporins, aminoglycosides, tetracyclines, polymyxins, macrolides, chloramphenicol, quinolones, and polypeptides. The individual substance might be, for example: rifaximine, neomycin sulphate, streptomycin sulphate, dihydrostreptomycin sulphate, tetracycline and its salts, oxytetracycline, chlortetracycline; penicillins, such as sodium penicillin, procaine penicillin, sodium ampicillin, benzatine penicillin, sodium cloxacillin, sodium dicloxacillin, a mixture of two penicillins such as sodium ampicillin and sodium cloxacillin or water-soluble salts of cephalosporin and benzatinic salts of cephalosporin.

These active constituents will be incorporated into the compound in proportion in such an amount as to enable their anti-bacterial potency to be effective. In vitro tests and in vivo tests have been carried out by means of compositions according to the invention.

## In vitro

An in vitro method was devised for evaluation of the rate at which the active constitutent of the various pharmaceutical compounds was released. The equipment used consisted of a 1000ml glass container, a heater with a magnetic stirrer, and a polythene cylinder 3cm in diameter.

The method envisaged warming 1000ml of full fat milk to 39 °C, and holding the temperature steady thermostatically, by way of the heater and stirrer.

The polythene cylinder was then immersed in the milk, and exactly 1 gram of the compound introduced into the cylinder. The milk stirring continuously, samples were taken at prescribed intervals of time.

Thus, the rate at which the active constituent was released could then be assessed by analyzing the samples. The reference product, a proprietory brand of the type mentioned in the preamble, contained the same antimicrobial agent (or agents) in the same proportions.

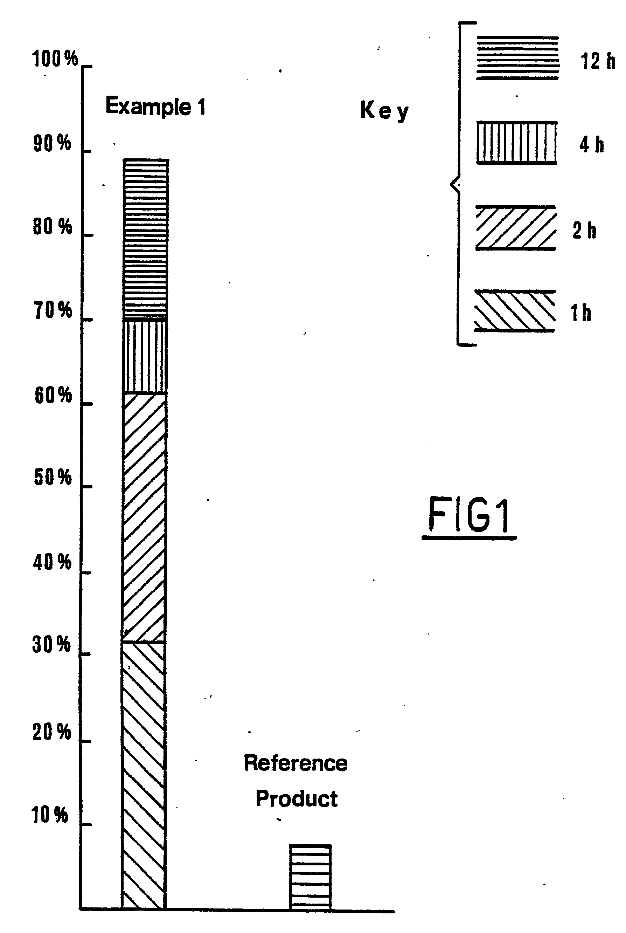

The results obtained are illustrated in fig 1 of the drawings, in which the column of example 1 shows the percentage rate at which the active constituent in the compounds disclosed was released over periods of 1, 2, 4 and 12 hours.

## In vivo

The results of the in vitro test were confirmed in vivo. Accurately measured quantities of the compound were administered, using the appropriate syringes and adopting the conventional procedure currently in use for antimastitis therapy carried out during lactation.

Samples of milk were taken at prescribed intervals, and a record was kept of the overall yield between samples. The rate at which the agent in the composition was released was then calculated and the results compared with those given by a reference product; here again, the reference was a proprietory formula containing the same active constituent.

The reference product was administered adopting the same methods and taking samples of milk at the same prescribed intervals.

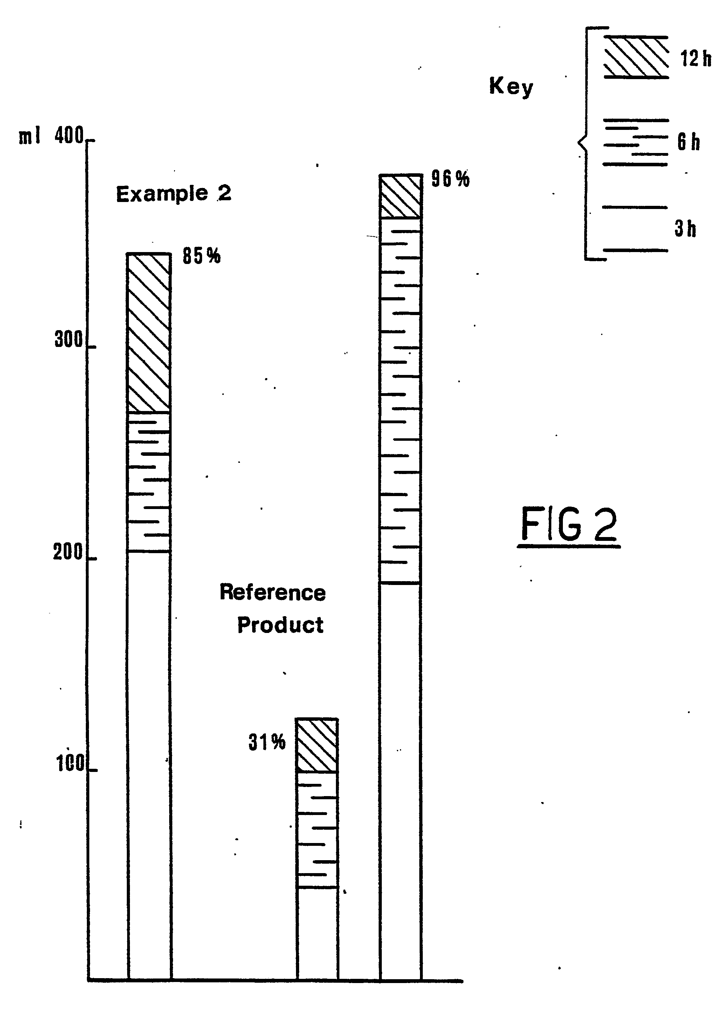

The results are illustrated in fig 2.

The right hand column refers to a solution of the agent in water injected into the udder, and shows the maximum therapeutic availability of the active constituent in percentage terms.

The reference product was dispersed in one of the conventional excipients in current use.

Calculation of residue

In determining the quantity of microbiologically active residual components active in the milk of the animals, individually and collectively, use was made of the agar disc method, as prescribed in the Italian Gazzetta Ufficiale (n° 194 - 1973).

The compounds listed below, and the method of their preparation, are given by way of examples:

| *Example 1 | grams |
|---|---|
| Sodium cloxacillin | 6 |
| Triglycerides of palmitic and stearic acid | 8 |
| Polyoxyethylenated cetyl and stearyl alcohol | 1.5 |
| Vaseline oil | 84.5 |

The vaseline oil, triglycerides of palmitic and stearyl acid, and polyoxyehylenated cetyl and stearyl alcohols are put into a turbo emulsifier, and melted at 140 °C. The resultant mass is cooled to 50 °C, whereupon the active constituent, sodium cloxacillin, is introduced and allowed to disperse, and the product is homogenized in a colloid mill. A smooth mass of creamy consistency is obtained. Proceeding as in example 1 above, though employing other antibiotics, the following compounds have been obtained.

```
*Example 2                                              grams

Sodium cephapirin                         :              6

Triglycerides of palmitic and stearic acid             10

Polyoxyethylenated cetyl and stearyl alcohol          2.5

Peanut oil                                            81.5

*Example 3                                              grams

Sodium ampicillin                                       4

Sodium dicloxacillin                                    4

Triglycerides of palmitic and stearic acid             8

Polyoxyethylenated cetyl and stearyl alcohol           5

Vaseline oil                              :            79

*Example 4                                             grams

Dihydrostreptomycin sulphate                          10

Sodium penicillin                                      7

Triglycerides of palmitic and stearic acid             8

Polyoxyethylenated cetyl and stearyl alcohol           3

Peanut oil                                            72

*Example 5                                             grams

Rifaximine                                             1

Triglycerides of palmitic and stearic acid            15

Polyoxyethylenated cetyl and stearyl alcohol           5

Vaseline oil                                          79

*Example 6                                             grams

Benzatine cephapirin                                 0.39

Triglycerides of palmitic and stearic acid            12

Polyoxyethylenated cetyl and stearyl alcohol           5

Peanut oil                                          82.61
```

## Claims

1. Veterinary composition for the prevention and cure of mastitis, intended for intramammary administration, characterized in that it contains at least one antimicrobial agent dispersed in a mixture consisting of triglycerides of palmitic and stearic acid with polyoxyethylenated cetyl alcohol and stearyl alcohol, and dispersed in an oily medium of mineral, vegetable, synthetic or mixed extraction.

2. Veterinary composition according to claim 1, wherein the antimicrobial agent is an antibiotic in form of a sulphamide, penicillin, cephalosporin, polymyxin, aminoglycoside, tetracycline, chloramphenicol, macrolide, quinolone and polypeptide.

3. Veterinary composition according to claim 1, wherein the antimicrobial agent is sodium cloxacillin, sodium ampicillin, sodium cephapirin, sodium dicloxacillin, sodium penicillin, neomycin sulphate, streptomycin sulphate dihydrostreptomycin sulphate, tetracycline, rifaximine, oxytetracycline or chlortetracycline.

4. Veterinary composition according to claims 1 and 3 which contains the antimicrobial agent in an amount of between 2 and 20% (W/W), the triglycerides of palmitic and stearic acid in an amount of between 0.5 and 20% (W/W), and the polyoxyethylenated cetyl and stearyl alcohol in an amount of between 0.5 and 20% (W/W).

5. Veterinary composition according to claims 1 to 4 in the form of a paste, cream or emulsion for the intramammary administration in antimastitic therapy conducted during lactation and dry periods.

**Patentansprüche**

1. Tierarzneimittel zur Vorbeugung gegen und Behandlung von Milchdrüsenentzündung mit Verabreichung direkt die durch Milchdrüsen, dadurch gekennzeichnet, daß es wenigstens einen antimikrobiellen Wirkstoff enthält, der in einer Mischung aus Palmin- und Stearinsäuretriglyzeriden mit polyoxyäthylenisiertem Cetyl- und Stearylalkohol dispergiert ist sowie in einem öligen Träger aus mineralischen, pflanzlichen, synthetischen oder gemischten Extrakten.

2. Tierarzneimittel nach Patentanspruch 1, dadurch gekennzeichnet, daß der antimikrobielle Wirkstoff ein Antibiotikum in Form von Sulphamiden, Penicillinen, Cephalosporinen, Polymyxinen, Aminoglykosiden, Chloramphenycolen, Makroliden, Chinolonen und Polypeptiden ist.

3. Tierarzneimittel nach Patentanspruch 1, dadurch gekennzeichnet, daß der antimikrobielle Wirkstoff aus Cloxacillinnatrium, Ampicillinnatrium, Dicloxacillinnatrium, Penicillinnatrium, Neomycinsulphat, Streptomycinsulphat, Dihydrostreptomycinsulphat, Tetrocyclin, Rifaximin, Oxytetracyclin oder Chlortetracyclin besteht.

4. Tierarzneimittel nach den Patentansprüchen 1 und 3, dadurch gekennzeichnet, daß der antimikrobielle Wirkstoff zu einem Anteil zwischen 2 und 20% (W/W), die Palmin- und Stearinsäuretriglyzeriden zu einem Anteil zwischen 0,5 und 20% (W/W) und die polyoxyäthylenisierten Cetyl- und Stearylalkohole zu einem Anteil zwischen 0,5 und 20% (W/W) enthält.

5. Tierarzneimittel nach den Patentansprüchen von 1 bis 4 in Form einer Paste, Salbe oder Emulsion zur Verabreichung direkt durch die Milchdrüse bei der Behandlung von Milchdrüsenentzündung während Säugezeit und der Trockenperiode.

**Revendications**

1. Composition vétérinaire pour la prévention et le traitement de la mastite, à administrer par voie intramammaire, caractérisée en ce qu'elle contient au moins un agent antimicrobien dispersé dans un mélange se composant de triglycérides de l'acide palmitique et stéarique, alcool cétylique et alcool stéarylique polyoxyétylénés, à son tour dispersé dans un véhicule huilleux d'extraction minérale, végétale, synthétique ou de synthèse.

2. Composition vétérinaire selon la revendication 1, caractérisée en ce que l'agent antimicrobien est un antibiotique sous forme de sulfamine, pénicilline, cephalosporine, polymyxine, aminoglycoside, tetracycline, macrolide, chloramphénycol, quinolone et polypeptide.

3. Composition vétérinaire selon la revendication 1, caractérisée en ce que l'agent antimicrobien est cloxacilline sodique, ampicilline sodique, céphapirine sodique, dicloxacilline sodique, pénicilline sodique, néomycine sulfate, streptomycine sulfate, dihydrostreptomycine sulfate, tetracycline, rifaximine, oxytétracycline ou chlortetracycline.

4. Composition vétérinaire selon les revendications 1 et 3, caractérisée en ce qu'elle contient l'agent antimicrobien dans une quantité de l'ordre de 2% (poids/poids) à 20% (poids/poids), les triglycérides de l'acide palmitique et stéarique dans une quantité de l'ordre de 0,5% (poids/poids) à 20% (poids/poids) et l'alcool cétylique et l'alcool stérylique polyoxyétylénés dans une quantité de l'ordre de 0,5% (poids/poids) à 20% (poids/poids).

5. Composition vétérinaire selon les revendications 1 à 4, caractérisée en ce qu'elle se présente sous forme de pommade, crème ou émulsion à administrer par voie intramammaire pour la thérapie contre la mastite effectuée pendant les périodes de lactation et de tarissement.

EP 0 222 712 B1

FIG1

FIG 2